# EUROPEAN PATENT APPLICATION

(11) **EP 4 483 812 A1**
(43) Date of publication of application: **01.01.2025**
(21) Application number: 23181482.3
(22) Date of filing: 26.06.2023
(51) Int. Cl.: A61B 6/03, A61B 6/00

(54) **CONTROL OF A COMPUTED TOMOGRAPHY SCANNER**

(71) Applicant: Koninklijke Philips N.V., 5656 AG Eindhoven (NL)
(72) Inventor: PROKSA, Roland, Eindhoven (NL); KOEHLER, Thomas, Eindhoven (NL)
(74) Representative: Philips Intellectual Property & Standards

(57) **Abstract**

Proposed are concepts pertaining to aiding and/or improving data acquisition for a CT scanner. In particular, embodiments of the invention propose concepts for modifying the data acquisition in such a way that the samples are directly on a parallel grid (in a r-φ sampling plot/diagram). This may reduce required processing effort/resource and may also avoid negative impacts of the re-binning processing (such as aliasing for example).

## Description

### FIELD OF THE INVENTION

The present invention relates to the field of Computer Tomography (CT) imaging, and in particular to the field of controlling a CT scanner.

### BACKGROUND OF THE INVENTION

In third generation CT systems, the x-ray source and the detector array are rotated with a gantry within the imaging plane and around the object to be imaged such that the angle at which the x-ray fan beam intersects the object constantly changes. A group of x-ray attenuation measurements (e.g., projection data), from the detector array at one gantry angle is referred to as a "view". A "scan" of the object comprises a set of views made at different gantry angles, or view angles, during one revolution of the x-ray source and detector about the object or patient being imaged.

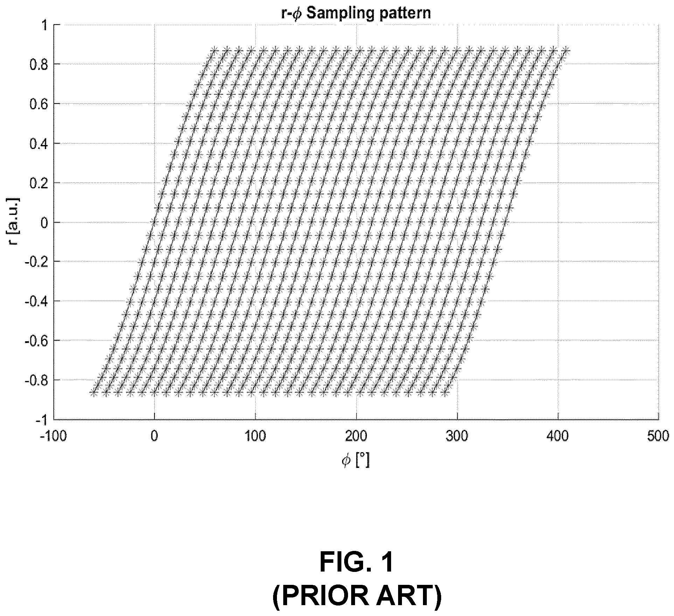

Many modem CT systems are helical scanners (also known as spiral scanners), in which the scanned object is continually moved while the projection data is being acquired. Such CT scanners typically have a so-called 'focus centered' cylindrical detector array (e.g. multiple rows/columns of detector pixels). This means that detector pixels are placed on a cylindrical shape with the X-ray focal spot being in the center. The geometry of the measured rays is often described in the so-called r-φ diagram/plot. In this r-φ diagram, the beam geometry of each individual pixel reading is marked with its distance to the center of rotation r and the beam angle φ (e.g., relative to the y-axis). Fig. 1 shows an exemplary r-φ sampling pattern, wherein the lines connecting the stars indicate pixel readings with identical gantry angle.

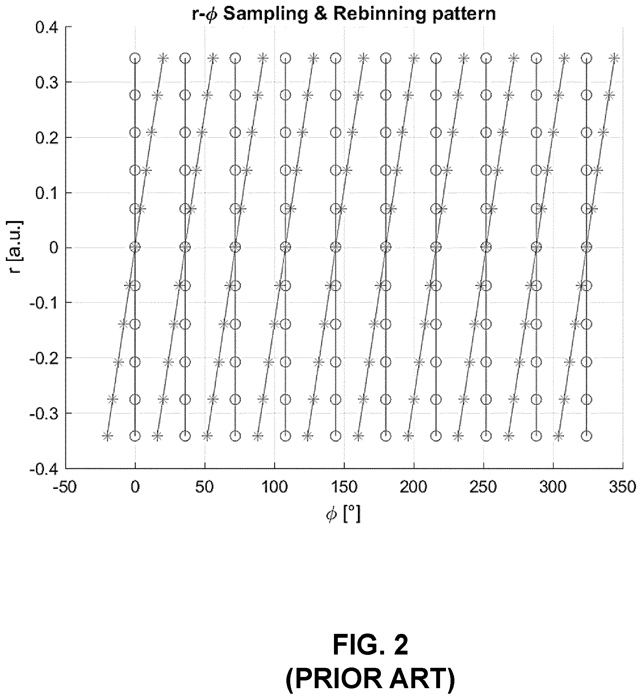

Typically, so-called `filtered back projection' methods are used for the image reconstruction. The back projection must take the geometry pattern as indicated in Fig. 1 to back project the pixel (filtered) readings. To do this, most reconstruction algorithms perform an interpolation from the measurement grid to the parallel beam grid before the filtration and back projection. This interpolation is called re-binning and consists of two interpolation steps: (i) an angular interpolation is done for the readings of each pixel in such a way that all readings fall onto an equidistant angular pattern; and (ii) the readings are interpolated in the r-direction to become equidistant. A close-up of the acquisition and re-binning pattern is shown in Fig. 2. The stars and connecting lines indicate the acquisition samples. The circles and lines indicate the target grid after angular re-binning. This first re-binning step is done pixel per pixel in angular direction. The second part of the re-binning process (not shown) interpolates the data on the vertical lines (along r) to get equidistant samples. The re-binning process is thus computationally expensive (i.e. requires significant processing effort/resource) and also has the drawback of introducing aliasing into the reconstructed image.

### SUMMARY OF THE INVENTION

The invention is defined by the claims.

According to examples in accordance with an aspect of the invention, there is provided a method for controlling data acquisition of a CT scanner with focus centered cylindrical detector array. The method comprises, for a plurality of columns of pixels of the detector array, controlling a timing of a data acquisition trigger signal for each pixel of the column so that a r-φ sampling pattern of the x-rays measured by the detector array is equivalent to a parallel beam geometry.

Proposed concepts thus aim to provide schemes, solutions, concepts, designs, methods and systems pertaining to aiding and/or improving data acquisition for a CT scanner. In particular, embodiments of the invention propose concepts for modifying the data acquisition in such a way that the samples are directly on a parallel grid (in a r-φ sampling plot/diagram). By way of example, it is proposed that this may be achieved by controlling the timing of the acquisition trigger pulses for each pixel (column) to be shifted (delayed) until the gantry rotation moves the pixel to the desired angle.

Embodiments are based on the realization that processing may be much easier and faster for equidistant sampling pattern (in r and φ), i.e. a so-called parallel beam geometry. This geometry is so much more attractive because, if data acquisition is performed directly on parallel angles (e.g. the circles in the diagram of Fig. 2), the first re-binning step can be omitted. This may reduce required processing effort/resource and may also avoid negative impacts of the re-binning processing (such as aliasing for example).

In other words, embodiments propose to control data acquisition of a CT scanner so that a r-φ sampling pattern of the detected x-rays exhibits a parallel beam geometry, thereby avoiding the need for a re-binning process to implemented for image reconstruction. Embodiments may therefore be used in relation to CT image processing so as to support a medical professional when selecting treatment for a subject. Such embodiments may also support clinical planning. Improved Clinical Decision Support (CDS) may therefore be provided by proposed concepts (e.g. by reducing or removing aliasing in acquired CT images).

In some embodiments, controlling a timing of a data acquisition trigger signal for a pixel comprises time-shifting the data acquisition trigger signal for the pixel based on a gantry angle (φ) of the pixel and a distance (r) of the pixel from the center of rotation of the cylindrical detector array. In this way, embodiments may obtain samples with a parallel beam geometry using a relatively simple/easy concept of controlling the timing of trigger signals for the CT scanner.

For example, time-shifting the data acquisition trigger signal for a pixel may comprise delaying the data acquisition trigger signal for the pixel until rotation of the gantry moves the pixel to a target gantry angle for the pixel.

Further, delaying the data acquisition trigger signal for the pixel may comprise determining a target time delay for the data acquisition trigger signal based on the target gantry angle (φ) for the pixel and a rotation speed of the gantry. In this way, the time delay may be aligned to the actual gantry rotation speed so as to achieve an angular shift (rather than a temporal shift).

In the parallel beam geometry, the pixel readings for a column of pixels of the detector may be at substantially the same projection angle.

In some embodiments, the CT scanner may comprise an angular trigger unit. Controlling a timing of a data acquisition trigger signal for a pixel may then comprise controlling an angular trigger unit of the CT scanner. Embodiments may therefore be used in conjunction with a known/existing angular trigger unit, which may have a precise and fast internal representation of the actual gantry angle.

According to another aspect, there is provided a computer program product for controlling data acquisition of a CT scanner with focus centered cylindrical detector array, wherein the computer program product comprises a computer-readable storage medium having computer-readable program code embodied therewith, the computer-readable program code configured to perform all of the steps of a proposed embodiment.

Thus, there may also be provided a computer system comprising a computer program product according to proposed embodiment; and one or more processors adapted to perform a method according to a proposed concept by execution of the computer-readable program code of said computer program product.

According to another aspect, there is provided a system for controlling data acquisition of a CT scanner with focus centered cylindrical detector array, the system comprising a controller configured to control, for a plurality of columns of pixels of the detector array, a timing of a data acquisition trigger signal for each pixel of the column so that an r-φ sampling pattern of the x-rays measured by the detector array is equivalent to a parallel beam geometry.

It will be understood that processing capabilities may be distributed throughout the system in different ways according to predetermined constraints and/or availability of processing resources.

Proposed embodiments may thus be employed in combination with conventional/existing CT acquisition/scanning systems. In this way, embodiments may integrate into legacy systems so as to improve and/or extend their functionality and capabilities. An improved CT imaging system may therefore be provided by proposed embodiments, for example.

Thus, there may be proposed concepts for improved data acquisition from a CT scanner. Such improved acquisition may, for instance, enable the first re-binning step to be omitted. By controlling data acquisition through the control of the timing of acquisition trigger pulses, an equidistant sampling pattern (in r and φ), i.e. parallel beam geometry, may be achieved and thus reduce required processing effort/resource for image reconstruction. The proposed concept(s) may also improve image quality (e.g. by reducing aliasing).

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an exemplary r-φ sampling pattern for conventional CT scanner data acquisition;
Fig. 2 is a close-up view of a conventional acquisition and re-binning pattern;
Fig. 3 is a simplified flow diagram of a method for controlling data acquisition of a CT scanner with focus centered cylindrical detector array according to an embodiment;
Fig. 4 depicts a simulation result of a modified forbild thorax phantom, wherein the right image depicts image obtained using conventional acquisition with 1200 readings per rotation. Level 0HU, window 50 HU, and wherein the left image depicts a corresponding image obtained using a proposed embodiment; and
Fig. 5 is a simplified block diagram of a computer within which one or more parts of an embodiment may be employed.

### DETAILED DESCRIPTION OF THE EMBODIMENTS

The invention will be described with reference to the Figures.

The invention proposes concepts for aiding and/or improving the acquisition of data from a CT scanner comprising a focus centered cylindrical detector array. In particular, embodiments of the invention may provide a method and/or system for controlling data acquisition of a CT scanner with focus centered cylindrical detector array, and this may reduce an amount of processing required for image construction. Improved image quality may also be obtained via a reduction in image aliasing (that would otherwise be caused by a first re-binning step that is avoid by proposed embodiments).

In particular, proposed concepts may provide an approach to modifying the data acquisition in such a way that the samples are directly on a parallel grid (in a r-φ sampling diagram/plot). Such modification may be by shifting (e.g. delaying) the acquisition trigger pulses for each pixel (column) until the gantry rotation moves the pixel to the desired angle.

Embodiments may therefore be particularly beneficial for reducing CT processing effort noise estimates, and such embodiments may achieve this by employing the proposed trigger pulse control concept(s). Embodiments may therefore be employed in, or in connection with, CT imaging systems that employ a focus centered cylindrical detector array.

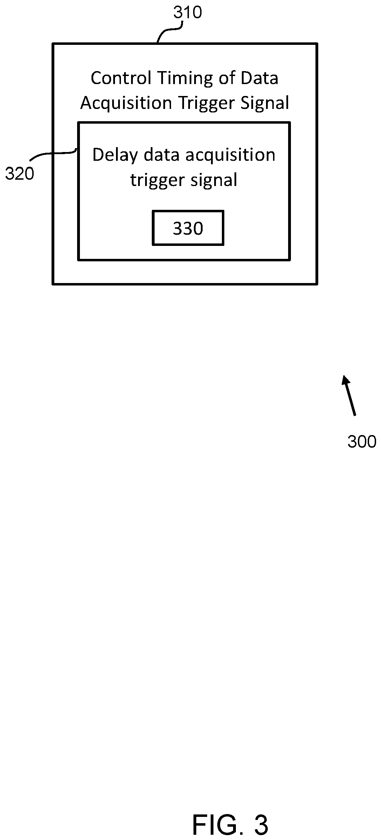

By way of example, an exemplary method for compressing a noise residuum will now be described with reference to Fig. 3.

Fig. 3 depicts a simplified flow diagram of a method 300 for controlling data acquisition of a CT scanner with focus centered cylindrical detector array according to an embodiment. Here, the CT scanner comprises a known angular trigger unit. More specifically, the CT scanner employs a trigger unit/system for operating the CT imaging system that models, by means of an adaptive digital phase-locked loop, A-DPLL, a gantry rotation of the gantry (wherein the A-DPLL is configured to minimize the difference between an actual gantry angle α(t)A and a modeled gantry angle α(t)M); and generates, for each of a plurality of predetermined values of the modeled gantry angle α(t)M, a trigger pulse for the detector. Such a concept/system for controlling a CT imaging system has been previously proposed by the applicant and is the subject of one or more existing patents and/or patent applications, including International Patent Application Number PCT/EP2022/086519.

The trigger unit has a precise and fast internal representation of the actual gantry angle. Its purpose is to generate just one precise trigger pulse for the entire detector. However, according to the proposed concept(s), this unit can be extended to generate individual trigger pulses to pixel columns aligned to the angular target pattern. This enables precise angular triggering virtually independent on the actual gantry speed. Put another way, the embodiment leverages a known trigger unit that allows for generating precise angular trigger pulses for high resolution CT imaging and high angular resolution by using an adaptive digital phase-locked loop which learns the angular pattern of the rotary encoder. The learned angular pattern may be used to correct trigger pulses for imaging.

The method 300 comprises, for a plurality of columns of pixels of the detector array, controlling 310 a timing of a data acquisition trigger signal for each pixel of the column so that an r-φ sampling pattern of the x-rays measured by the detector array is equivalent to a parallel beam geometry. In the parallel beam geometry, the pixel readings for a column of pixels of the detector are at substantially the same projection angle. Also, such controlling 310 comprises controlling the angular trigger unit of the CT scanner.

More specifically, step 310 of controlling a timing of a data acquisition trigger signal for a pixel comprises time-shifting the data acquisition trigger signal for the pixel based on a gantry angle (φ) of the pixel and a distance (r) of the pixel from the center of rotation of the cylindrical detector array.

By way of example, time-shifting the data acquisition trigger signal for a pixel comprises delaying 320 the data acquisition trigger signal for the pixel until rotation of the gantry moves the pixel to a target gantry angle for the pixel. This step of delaying 320 the data acquisition trigger signal includes the step of determining 330 a target time delay for the data acquisition trigger signal based on the target gantry angle (φ) for the pixel and a rotation speed of the gantry. In this way, the pixel dependent acquisition trigger employs a time shift relative to the trigger of other pixels. This time delay is aligned to the actual gantry rotation speed (because the purpose is to have an angular shift instead of a temporal shift).

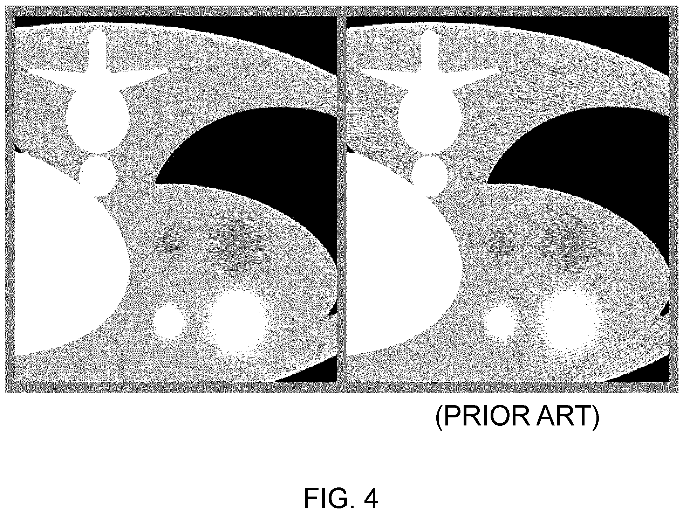

The potential image quality improvement facilitated by the proposed concept(s) is illustrated in Fig. 4.

Fig. 4 depicts a simulation result of a modified forbild thorax phantom, wherein the right image in Fig. 4 depicts image obtained using conventional acquisition with 1200 readings per rotation. Level 0HU, window 50 HU, and wherein the left image in Fig. 4 depicts a corresponding image obtained using the proposed control concept(s).

In this exemplary simulation, a single focal spot acquisition with 1200 readings per rotation was simulated. From Fig. 4, it can be seen that the proposed acquisition concept suffers less from angular aliasing than the standard/conventional acquisition scheme.

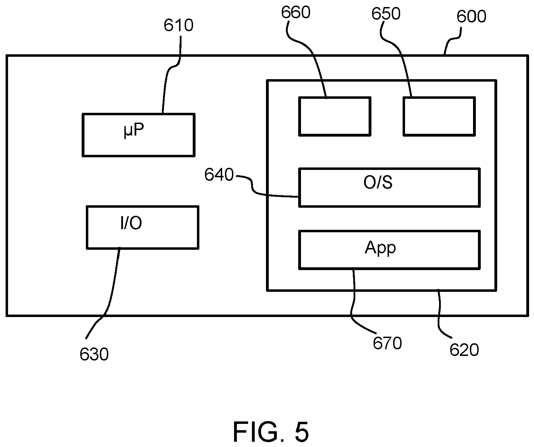

Fig. 5 illustrates an example of a computer 600 within which one or more parts of an embodiment may be employed. Various operations discussed above may utilize the capabilities of the computer 600. For example, one or more parts of the proposed CT scanner control system may be incorporated in any element, module, application, and/or component discussed herein. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620, and one or more I/O devices 670 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor-based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 640, source code 630, and one or more applications 660 in accordance with exemplary embodiments. As illustrated, the application 660 comprises numerous functional components for implementing the features and operations of the exemplary embodiments. The application 660 of the computer 600 may represent various applications, computational units, logic, functional units, processes, operations, virtual entities, and/or modules in accordance with exemplary embodiments, but the application 660 is not meant to be a limitation.

The operating system 650 controls the execution of other computer programs, and provides scheduling, input-output control, file and data management, memory management, and communication control and related services. It is contemplated by the inventors that the application 660 for implementing exemplary embodiments may be applicable on all commercially available operating systems.

Application 660 may be a source program, executable program (object code), script, or any other entity comprising a set of instructions to be performed. When a source program, then the program is usually translated via a compiler (such as the compiler 640), assembler, interpreter, or the like, which may or may not be included within the memory 620, so as to operate properly in connection with the O/S 650. Furthermore, the application 660 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 670 may include input devices such as, for example but not limited to, a mouse, keyboard, scanner, microphone, camera, etc. Furthermore, the I/O devices 670 may also include output devices, for example but not limited to a printer, display, etc. Finally, the I/O devices 670 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 670 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at startup, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 660 and the O/S 650 are read, in whole or in part, by the processor 610, perhaps buffered within the processor 610, and then executed.

When the application 660 is implemented in software it should be noted that the application 660 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 660 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The method of Fig. 1 may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

Data describing an alertness value determined according to an embodiment may be stored on a storage medium. Task execution data according to an embodiment may be stored on the same storage medium or a different storage medium. Such data may be transmitted as a signal modulated onto an electromagnetic carrier wave. The signal may be defined according to a standard for digital communications. The carrier wave may be an optical carrier, a radiofrequency wave, a millimeter wave, or a near field communications wave. It may be wired or wireless.

To the extent that an embodiment is implemented partly or wholly in hardware, the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Also, reference to columns of pixels of a detector array should also be taken to refer to rows of pixels of a detector. That is, columns may be rows, depending on viewing direction for example.

## Claims

1. A method (300) for controlling data acquisition of a CT scanner with focus centered cylindrical detector array, the method comprising:
for a plurality of columns of pixels of the detector array, controlling (310) a timing of a data acquisition trigger signal for each pixel of the column so that an r-φ sampling pattern of the x-rays measured by the detector array is equivalent to a parallel beam geometry.

2. The method of claim 1, wherein controlling (310) a timing of a data acquisition trigger signal for a pixel comprises time-shifting the data acquisition trigger signal for the pixel based on a gantry angle (φ) of the pixel and a distance (r) of the pixel from the center of rotation of the cylindrical detector array.

3. The method of claim 2, wherein time-shifting the data acquisition trigger signal for the pixel comprises:
delaying (320) the data acquisition trigger signal for the pixel until rotation of the gantry moves the pixel to a target gantry angle for the pixel.

4. The method of claim 3, wherein delaying the data acquisition trigger signal for the pixel comprises:
determining (330) a target time delay for the data acquisition trigger signal based on the target gantry angle (φ) for the pixel and a rotation speed of the gantry.

5. The method of any of claims 1 to 3, wherein, in the parallel beam geometry, the pixel readings for a column of pixels of the detector are at substantially the same projection angle.

6. The method of any of claims 1 to 5, wherein the CT scanner comprises an angular trigger unit, and wherein controlling a timing of a data acquisition trigger signal for a pixel comprises controlling an angular trigger unit of the CT scanner.

7. A computer program comprising computer program code means adapted, when said computer program is run on a computer, to implement the method of any of claims 1 to 6.

8. A system for controlling data acquisition of a CT scanner with focus centered cylindrical detector array, the system comprising:
controller configured to control, for a plurality of columns of pixels of the detector array, a timing of a data acquisition trigger signal for each pixel of the column so that an r-φ sampling pattern of the x-rays measured by the detector array is equivalent to a parallel beam geometry.

9. The system of claim 8, wherein the controller is configured to time-shift the data acquisition trigger signal for a pixel based: on a gantry angle (φ) of the pixel and a distance (r) of the pixel from the center of rotation of the cylindrical detector array.

10. The system of claim 9, wherein time-shifting the data acquisition trigger signal for the pixel comprises:
delaying the data acquisition trigger signal for the pixel until rotation of the gantry moves the pixel to a target gantry angle for the pixel.

11. The system of claim 10, wherein delaying the data acquisition trigger signal for the pixel comprises:
determining a target time delay for the data acquisition trigger signal based on the target gantry angle (φ) for the pixel and a rotation speed of the gantry.

12. The system of any of claims 8 to 11, wherein, in the parallel beam geometry, the pixel readings for a column of pixels of the detector are at substantially the same projection angle.

13. A CT imaging system comprising:
a CT scanner with focus centered cylindrical detector array; and
a system for controlling data acquisition of the CT scanner according to any of claims 8 to 12,
wherein the CT scanner comprises an angular trigger unit,
and wherein the controller is configured to control an angular trigger unit of the CT scanner.
